# EUROPEAN PATENT APPLICATION

(11) **EP 1 055 732 A1**
(43) Date of publication of application: **29.11.2000**
(21) Application number: 99959901.2
(22) Date of filing: 20.12.1999
(51) Int. Cl.: C12P 7/22, C12N 9/02

(54) **PROCESS FOR PRODUCING (R)-2-HYDROXY-1-PHENOXYPROPANE DERIVATIVE**

(30) Priority: 18.12.1998 JP 35990298
(71) Applicant: KANEKA CORPORATION, Osaka-shi, Osaka 530-8288 (JP)
(72) Inventor: TAOKA, Naoaki, Kobe-shi, Hyogo 654-0122 (JP); KIZAKI, Noriyuki, Kakogawa-shi, Hyogo 675-0057 (JP); YASOHARA, Yoshihiko, Himeji-shi, Hyogo 670-0942 (JP); INOUE, Kenji, Kakogawa-shi, Hyogo 675-0039 (JP); HASEGAWA, Junzo, Akashi-shi, Hyogo 674-0057 (JP)
(74) Representative: Hubert, Philippe
(86) International application number: JP9907127
(87) International publication number: WO0037666

(57) **Abstract**

The present invention is related to producing (R)-2-hydroxy-1-phenoxypropane derivatives, which are of value as intermediates of pharmaceuticals, with high optical purity and good efficiency,
comprising a step of reducing a phenoxyacetone derivative with a carbonyl reductase derived from a microorganism of the genus Candida or a culture broth or processed culture broth of said microorganism having the ability to elaborate the carbonyl reductase.

## Description

### TECHNICAL FIELD

The present invention relates to a process for producing (R)-2-hydroxy-1-phenoxypropane derivatives which comprises reducing phenoxyacetone derivatives R-selectively with a microorganism or the like. (R)-2-hydroxy-1-phenoxypropane derivatives such as, inter alia, 3,4-difluoro-2-((R)-2-(hydroxypropyloxy))nitrobenzene are compounds of use as intermediates of synthetic antimicrobials.

### BACKGROUND ART

While several technologies have heretofore been reported for the microbiological reduction of phenoxyacetone derivatives, it is known that, when bakers' yeast, for instance, is employed, the S-compounds are preferentially produced as generally accounted for by the Prelog rule (Acta. Chem. Scand., 48 (6), 506, 1994). Thus, microorganisms known to have the ability to reduce phenoxyacetone derivatives with R-selectivity are very few and there has not been found a microorganism capable of achieving strictly R-selective reduction as yet. For example, the technology for R-selective reduction which comprises using a microorganism of the genus Thermoaerobacter is known but the optical purity of the product is 90% ee at most (Japanese Kokai Publication Hei-8-266292). The technology for R-selective reduction of 2-acetonyloxy-3,4-difluoronitrobenzene which comprises using a microorganism of the genus Corynebacterium is also known but the optical purity of the product is only about 88.4% ee (Japanese Kokai Publication Hei-5-68577). Also known is the technology for R-selective reduction of 2-acetonyloxy-3,4-difluoronitrobenzene which comprises using a fungal strain but the optical purity of the product is only about 84 to 94% ee and, in addition, the substrate concentration that can be used is so low that the productivity is also inevitably low (Japanese Kokai Publication Hei-3-183489). When microbial cells are used, the presence of S-selective carbonyl reductase in the cell tends to detract from the optical purity of the product. Therefore, the technology for R-selective reduction of phenoxyacetone or the like which comprises the use of a purified R-selective alcohol dehydrogenase, a coenzyme and an enzyme necessary to regenerate the coenzyme has been developed. However, this technology cannot be said to be practically useful, partly because the optical purity of the product is not sufficiently high and partly because the technology requires a costly purified enzyme (USP 5385833).

Thus, those reduction technologies utilizing a microorganism or the like invariably have room for improvement in order that they may be successfully exploited on a commercial scale because 1) the optical purity of the product is not as high as required of any starting material for pharmaceuticals, 2) the low substrate concentration entails low productivity, and/or 3) a costly purified enzyme is required.

### SUMMARY OF THE INVENTION

The inventors of the present invention conducted intensive investigations for producing (R)-2-hydroxy-1-phenoxypropane derivatives, which are of value as intermediates of pharmaceuticals, especially 3,4-difluoro-2-((R)-2-(hydroxypropyloxy))nitrobenzene which is of use as an intermediate of synthetic antimicrobials, with high optical purity and good efficiency and discovered a carbonyl reductase capable of reducing phenoxyacetone derivatives, which are available at low cost, to (R)-2-hydroxy-1-phenoxypropane derivatives with high R-selectivity and good efficiency. The present invention has been accordingly developed.

The present invention, therefore, is directed to a process for producing an (R)-2-hydroxy-1-phenoxypropane derivative of the general formula (1): 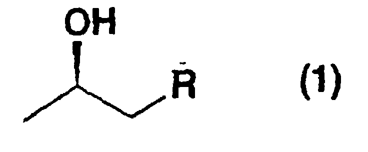 wherein R represents a phenoxy group or a substituted phenoxy group,
which comprises a step of reducing a phenoxyacetone derivative of the general formula (2): 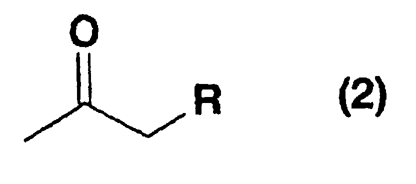 wherein R is as defined above,
with a carbonyl reductase derived from a microorganism of the genus Candida or a culture broth or processed culture broth of a microorganism having the ability to elaborate said carbonyl reductase, and a step of recovering the product (R)-2-hydroxy-1-phenoxypropane derivative of said general formula (1).

The present invention is now described in detail.

### DETAILED DESCRIPTION OF THE INVENTION

Referring to the above formulas (1) and (2), R represents a phenoxy group or a substituted phenoxy group, and the substituted phenoxy group includes p-nitrophenoxy, p-aminophenoxy, p-chlorophenoxy, p-bromophenoxy, 3,5-dinitrophenoxy, 2,3-difluoro-5-nitrophenoxy, etc. and is preferably 2,3-difluoro-5-nitrophenoxy.

Referring to the phenoxyacetone derivative of the general formula (2) which is used as the starting material in the present invention, it is known that 2-acetonyloxy-3,4-difluoronitrobenzene, for instance, can be easily synthesized by reacting 2,3-difluoro-5-nitrophenol with chloroacetone in the presence of a strong base (Japanese Kokai Publication Sho-61-246151).

As the carbonyl reductase capable of R-selective reduction, there can be mentioned the enzyme derived from a microorganism of the genus Candida, preferably Candida magnoliae, more preferably Candida magnoliae IFO0705.

The microorganism capable of elaborating said enzyme may be whichever of a wild strain and a mutant strain. The microorganism obtainable by a genetic engineering technique such as cell fusion or gene manipulation can also be employed. Such genetically engineered producer strain can be typically acquired by the method which comprises a step of isolating and/or purifying the particular enzyme and determining either the whole or a part of its amino acid sequence, a step of acquiring a DNA sequence encoding said enzyme based on said amino acid sequence, a step of introducing this DNA into a different host microorganism to construct a recombinant strain of microorganism, and a step of growing the recombinant strain to let it produce the objective enzyme (WO 98-35025). By way of example, there can be mentioned the transformant cell line obtainable by such a transformation using a plasmid, said plasmid containing a DNA encoding for said carbonyl reductase and a DNA encoding for an enzyme capable of regenerating the coenzyme on which said carbonyl reductase is dependent. The preferred, among such transformant cell lines, are the transformant cell line in which said enzyme capable of regenerating the coenzyme is glucose dehydrogenase, the transformant cell in which said glucose dehydrogenase is derived from Bacillus megaterium, the transformant cell line in which said plasmid is pNTS1G, and the transformant cell line obtained by the transformation of an Escherichia coli strain. The more preferred example is E. coli HB101 (pNTS1G), Accession No. FERM-BP5835 [deposited with: National Institute of Bioscience and Human Technology, Agency of Industrial Science and Technology, the Ministry of International Trade and Industry, Japan (NIBH, Higashi 1-1-3, Tsukuba-shi, Ibaraki, Japan); date of acceptance: February 24, 1997].

In the production technology according to the present invention, the culture medium for the microorganism capable of elaborating said carbonyl reductase is not particularly restricted only provided that the microorganism may grow thereon. For example, the ordinary liquid medium containing various carbon sources such as carbohydrates, e.g. glucose, sucrose, etc., alcohols, e.g. ethanol, glycerol, etc., fatty acids, e.g. oleic acid, stearic acid, etc., inclusive of the corresponding esters, oils, e.g. rapeseed oil, soybean oil, etc.; nitrogen sources such as ammonium sulfate, sodium nitrate, peptone, casamino acids, corn steep liquor, wheat bran, yeast extract, etc.; inorganic salts such as magnesium sulfate, sodium chloride, calcium carbonate, potassium monohydrogenphosphate, potassium dihydrogenphosphate, etc.; and other nutrients such as malt extract, meat extract, etc. can be employed. Cultivation is carried out under aerobic conditions, and the cultural time is usually about 1 to 5 days, the medium pH is 3 to 9, and the incubation temperature is 10 to 50 °C.

The reaction can be conducted by adding the substrate phenoxyacetone derivative, coenzyme NADP, and a culture broth or processed culture broth of a microorganism to a suitable solvent and stirring the mixture under controlled pH condition. The reaction is carried out at 10 to 70 °C and pH 4 to 10. The substrate charge concentration is 0.1 to 90% (w/v) and the substrate can be added in bolus or continuously. This reaction can be carried out batchwise or continuously.

The term "culture broth of a microorganisms" as used herein means a culture broth inclusive of cells or a cellular fraction thereof (cultured cells), and the "processed culture broth thereof" includes a crude extract, lyophilized cells, acetone-dehydrated cells, and a homogenate of such cells. Furthermore, such enzymes or cells can be used as immobilized by any known technique in advance. Such immobilization can be effected by procedures well known in the art (e.g. crosslinking, physical adsorption, entrapment, etc.).

In conducting the reaction, the amount of the expensive coenzyme can be drastically reduced by using a conventional NADPH regeneration system in combination. The representative NADPH regeneration system is that of glucose dehydrogenase and glucose. While the reaction conditions depend on the particular enzyme, microorganism, or culture broth or processed culture broth as well as on substrate concentration, generally the substrate concentration is about 0.1 to 90 weight %, the reaction temperature is 10 to 50 °C, the reaction pH is 5 to 8, and the reaction time is 1 to 36 hours.

The objective (R)-2-hydroxy-1-phenoxypropane derivative can be produced at reduced cost by conducting the above reaction using a culture broth or processed culture broth of a transformant, said transformant being obtained by introducing, into a homologous host microorganism, the carbonyl reductase gene and the gene of the enzyme (e.g. glucose dehydrogenase) capable of regenerating the coenzyme on which said carbonyl reductase is dependent, for it will then be unnecessary to provide an enzyme precursor necessary to generate the coenzyme.

The (R)-2-hydroxy-1-phenoxypropane derivative formed as the result of said reaction can be purified by the routine procedure. For example, when a microorganism or the like has been used, the (R)-2-hydroxy-1-phenoxypropane derivative can be purified by removing the suspended matter inclusive of cells by centrifugation or filtration where necessary, extracting the supernatant or filtrate with an organic solvent such as ethyl acetate, toluene or the like, removing the organic solvent under reduced pressure, and subjecting the residue to distillation under reduced pressure, chromatography or the like.

### BEST MODE FOR CARRYING OUT THE INVENTION

The following examples illustrate the present invention in further detail without defining the scope of the invention.

### Example 1

A 500-ml Sakaguchi flask containing 100 ml of a semi-synthetic medium (glycerin 15 g, yeast extract 3 g, Na₂HPO₄ 6 g, KH₂HPO₄ 3 g, NaCl 2 g, MgSO₄·7H₂O 0.5 g, water 1 L, pH=7.2) after sterilization was inoculated with the recombinant E. coli HB101 (pNTS1G) FERM-BP5835, and shake culture was carried out at 37 °C for 28 hours. To 50 ml of the culture broth obtained were added phenoxyacetone 5 g, glucose 9 g, and NADP 4.4 mg, and the reaction was carried out at 30 °C under constant stirring for 24 hours, with the reaction system being controlled at pH=6.5 with 5 N-aqueous sodium hydroxide solution. After completion of the reaction, the reaction mixture was extracted with toluene and the extract was concentrated with reduced pressure to recover (R)-2-hydroxy-1-phenoxypropane as a residue. HPLC analysis of the reaction mixture [column: FinepakC_{18:5} (Nippon Bunko), eluent: acetonitrile/0.1% phosphoric acid = 30/70, flow rate: 0.7 ml/min, detection wavelength: 210 nm] revealed that the conversion rate was 79%. A portion of the above concentration residue was purified by silica gel column chromatography and analyzed by HPLC [column: Chiralcel AS (Daicel Chemical), eluent: hexane/isopropyl alcohol = 98/2, flow rate: 1 ml/min, temperature: 40 °C, detection wavelength: 210 nm]. The optical purity was 99.7% ee.

### Example 2

To 50 ml of the culture broth obtained in Example 1 were added 2-acetonyloxy-3,4-difluoronitrobenzene 2.5 g, glucose 2.97 g and NADP 2.9 mg, and the reaction was carried out at 30 °C under constant stirring for 23 hours, with the reaction system being controlled at pH=6.5 with 5 N-aqueous sodium hydroxide solution. After completion of the reaction, the reaction mixture was extracted with toluene and the extract was concentrated with reduced pressure to recover 3,4-difluoro-2-((R)-2-(hydroxypropyloxy))nitrobenzene as a residue. HPLC analysis of the reaction mixture (under the same conditions as in Example 1) revealed that the conversion rate was 80%. A portion of the above concentration residue was purified by column chromatography and, after dinitrobenzoylation, HPLC analysis (column: Chiralcel OD-H (Daicel Chemical), eluent: hexane/isopropyl alcohol = 50/50, flow rate: 1 ml/min, temperature: 40 °C, detection wavelength: 254 nm) was performed. The optical purity was not less than 99% ee.

### INDUSTRIAL APPLICABILITY

According to the process of the present invention, (R)-2-hydroxy-1-phenoxypropane derivatives which are of great use as intermediates of pharmaceuticals can be produced with high optical purity and good efficiency.

## Claims

1. A process for producing an (R)-2-hydroxy-1-phenoxypropane derivative of the general formula (1): 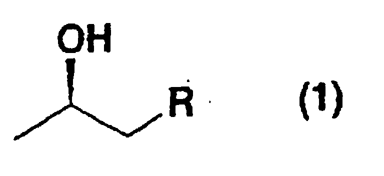 wherein R represents a phenoxy group or a substituted phenoxy group,
which comprises a step of reducing a phenoxyacetone derivative of the general formula (2): 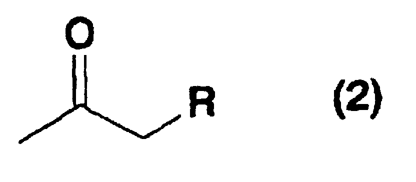 wherein R is as defined above,
with a carbonyl reductase derived from a microorganism of the genus Candida or a culture broth or processed culture broth of a microorganism having the ability to elaborate said carbonyl reductase and
a step of recovering the product (R)-2-hydroxy-1-phenoxypropane derivative of said general formula (1).

2. The process for producing according to Claim 1
wherein R is 2,3-difluoro-5-nitrophenoxy group.

3. The process for producing according to Claim 1 or 2
wherein said microorganism of the genus Candida is Candida magnoliae.

4. The process for producing according to Claim 3
wherein said microorganism of the genus Candida is Candida magnoliae IFO0705.

5. The process for producing according to Claim 1 or 2
wherein said microorganism having the ability to elaborate said carbonyl reductase derived from a microorganism of the genus Candida is a transformant cell line obtainable by a transformation using a plasmid,
said plasmid containing a DNA encoding said carbonyl reductase and a DNA encoding an enzyme capable of regenerating a coenzyme on which said carbonyl reductase is dependent.

6. The process for producing according to Claim 5
wherein said enzyme capable of regenerating a coenzyme is a glucose dehydrogenase.

7. The process for producing according to Claim 6
wherein said glucose dehydrogenase is derived from Bacillus megaterium.

8. The process for producing according to any of Claims 5 to 7
wherein said carbonyl reductase is derived from Candida magnoliae.

9. The process for producing according to Claim 8
wherein said carbonyl reductase is derived from Candida magnoliae IFO0705.

10. The process for producing according to any of Claims 5 to 9
wherein said plasmid is pNTS1G.

11. The process for producing according to any of Claims 5 to 10
wherein said transformant cells are transformant Escherichia coli cells.

12. The process for producing according to Claim 11
wherein said transformant cells are said transformant E. coli HB101 (pNTS1G) FERM-BP5835 cells.
